# FASCICULE DE BREVET EUROPEEN

(11) **EP 0 355 102 B1**
(45) Date de publication et mention de la délivrance du brevet: **20.10.1993**
(21) Numéro de dépôt: 88903293.4
(22) Date de dépôt: 15.04.1988
(51) Int. Cl.: C12P 1/00, C12P 41/00, C12P 7/40, C12P 7/50

(54) **PROCEDE ELECTROENZYMATIQUE DE PRODUCTION DE COMPOSES DE PURETE ENANTIOMERIQUE CONTROLEE**
ELEKTROENZYMATISCHES VERFAHREN ZUR HERSTELLUNG VON VERBINDUNGEN MIT KONTROLLIERTER ENANTIOMERISCHER REINHEIT
ELECTROENZYMATIC METHOD FOR PRODUCING COMPOUNDS HAVING A CONTROLLED ENANTIOMERIC PURITY

(30) Priorité: 17.04.1987 FR 8705547
(43) Date de publication de la demande: 28.02.1990
(73) Titulaire: CENTRE NATIONAL DE LA RECHERCHE SCIENTIFIQUE (CNRS), 75700 Paris Cedex 07 (FR)
(72) Inventeur: BOURDILLON, Christian, F-60880 Le Meux (FR); MOIRAUX, Jacques, F-75012 Paris (FR); BONNEFOY, Jacques, Philippe, Bernard, F-75007 Paris (FR); LAVAL, Jean-Marc, F-60200 Compiègne (FR)
(74) Mandataire: Phélip, Bruno
(86) Numéro de dépôt international: FR8800189
(87) Numéro de publication internationale: WO8808029

(56) Documents cités:
- EP-A- 0 030 195
- EP-A- 0 099 742
- EP-A- 0 138 040
- EP-A- 0 198 440
- WO-A-80/00453
- WO-A-82/03729

## Description

La présente invention concerne, d'une façon générale, la mise en oeuvre de réactions d'oxydation et de réduction de molécules organiques, dans un procédé combinant des réactions électrochimiques et des catalyses enzymatiques, et, en particulier, un procédé électroenymatique de production d'un composé de pureté énantiomérique contrôlée.

Les échanges d'électrons sont catalysés dans les organismes vivants par des enzymes nommées oxydoréductases. Ces enzymes sont particulièrement intéressantes en technologie enzymatique, car elles catalysent des réactions d'oxydation ou de réduction partielles. On peut mentionner, à titre d'exemple de ces enzymes, les déshydrogénases, et, notamment, les déshydrogénases à co-substrat du type Nicotinamide Adénine Dinucléotide (ci-après désigné par l'abréviation NAD) ou Nicotinamide Adénine Dinocléotide Phosphate (ci-après désigné par l'abréviation NADP), lesquelles sont des catalyseurs très performants par leur spécificité et leur sélectivité. Elles catalysent des réactions réversibles du type : 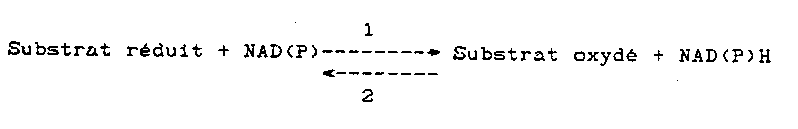
Les substrats concernés portent, en règle générale, des groupements plus ou moins oxygénés qui sont modifiés par la réaction.

La mise en pratique de tout procédé du type précité suppose que l'on puisse recycler le co-substrat, à savoir, dans l'exemple susmentionné, le NAD (P) si l'on désire utiliser le sens 1 de la réaction ci-dessus, le bilan étant une oxydation du substrat réduit, ou bien le NAD(P)H, si l'on désire utiliser le sens 2 de la réaction ci-dessus, le bilan étant une réduction du substrat oxydé. En effet, le NAD et le NADP sont des molécules dont le coût est tel qu'il faut pouvoir les régénérer cycliquement plusieurs milliers de fois pour que le procédé soit économiquement acceptable.

Parmi les différentes méthodes de régénération (chimique, enzymatique, microbiologique et électrochimique), la régénération enzymatique, suivant laquelle on utilise une deuxième enzyme, ainsi que d'autres substrats, pour effectuer la régénération, est actuellement considérée comme étant la plus performante en capacité de recyclage. Toutefois, elle présente l'inconvénient de compliquer le procédé, en particulier parce qu'elle implique différentes étapes de séparation. En revanche, la régénération éléctrochimique est particulièrement attrayante de ce point de vue, car il ne se forme aucun sous-produit ; cependant, la régénération électrochimique est encore considérée dans la littérature comme étant peu performante.

Dans le cadre des études qui ont conduit à la présente invention, on a constaté que, contrairement à ce préjugé, l'oxydation électrochimique du NADH en NAD peut être obtenue avec un très haut rendement (supérieur à 99,99%), ce que permet d'obtenir au minimum 10 000 cycles de régénération. Il en résulte que la voie directe de l'oxydation d'un substrat (sens 1 du schéma réactionnel ci-dessus), utilisant des déshydrogénases, est applicable indutriellement par le procédé enzymatique.

En revanche, la réduction électrochimique directe du NAD en NADH conduit à un nombre de cycles de régénération très faible, inférieur à 10, et, en conséquence, il n'est pas possible de faire appel à cette technique dans une application effectuée a l'échelle industrielle. Or, du point de vue économique, c'est précisément le sens de réduction (sens 2 du schéma réactionnel ci-dessus) qui est intéressant, car il conduit à des composés optiquement actifs qui présentent une pureté élevée et qui sont difficiles à préparer par des méthodes purement chimiques.

A titre d'exemple, on peut citer la réduction chimique ou électrochimique d'une cétone non symétrique, qui conduit à un mélange racémique, difficile à purifier, de steréoisomères D et L, en règle générale, en des quantités équimoléculaires.

Comme on l'a indiqué ci-dessus, la présente invention s'intéresse à la production de ces composés optiquement actifs. Dans ce qui suit, on désignera par S, un substrat de départ, et par P, le produit d'arrivée, qui porte un carbone asymétrique. P peut se trouver sous deux formes isomériques optiquement actives notées respectivement D-P et L-P. Le mélange des deux formes (racémique) est noté D/L-P.

Le problème à résoudre est celui d'obtenir, à partir de S (ou de de D/L-P ou encore d l'isomére optique inverse de celui que l'on veut préparer) des composés réduits L-P ou D-P, de pureté énantiomérique contrôlée, sans avoir besoin de mettre en oeuvre de réduction électrochimique du NAD ou autre co-substrat de l'oxydoréductase utilisée.

La solution apportée par la présente invention repose sur l'utilisation simultanée [1] de la réduction électrochimique non stéréospécifique du substrat S, conduisant au mélange racémique D/L-P, et [2] de l'oxydation inverse catalysée par l'enzyme oxydoréductase et stéréospécifique, conduisant au substrat S recycle dans la réduction électrochimique non stéréospécifique, le co-substrat de l'enzyme étant avantageusement régénéré par oxydation électrochimique. Dans ces conditions, chaque cycle de S provoque un enrichissement en l'isomère qui n'a pas été impliqué dans l'oxydation stéréospécifique.

Le schéma réactionnel des deux réactions principales [1] et [2], partiellement antagonistes, et de l'oxydation électrochimique du co-substrat de l'enzyme utilisée, dans le cas où celle-ci est la L-déshydrogénase dont le co-substrat est NAD, est le suivant : 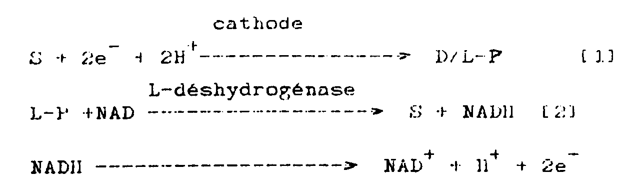
Dans ce cas, l'isomère D-P n'étant pas transformé, chaque cycle de S provoque un enrichissement en D-P. De la même manière, en choisissant une D-déshydrogénase, ou peut enrichir le système en isomère L-P.

La présente invention a donc d'abord pour objet un procédé électroenzymatique de production d'un composé de pureté énantiomérique contrôlée, respectivement D-P ou L-P, à partir d'un substrat S constitué par la forme oxydée du racémique correspondant D/L-P, ou dudit racémique D/L-P, ou encore de l'isomère optique inverse de celui que l'on veut préparer, caractérisé par le fait qu'on introduit, dans un réacteur électrochimique, respectivement ledit substrat S, ledit racémique D/L-P ou l'isomère optique inverse de celui que l'on veut préparer, ainsi qu'une enzyme oxydoréductase capable de catalyser l'oxydation de l'isomère optique inverse de celui que l'on veut préparer ; qu'on impose une différence de potentiel entre les électrodes, afin d'effecteur la réduction cathodique non stéréospécifique dudit substrat S, jusqu'a obtention de l'isomère ayant la pureté énantiomérique recherchée ; et qu'on régénère le co-subtrat de ladite enzyme par oxydation anodique dans ledit réacteur électrochimique.

Ce procédé s'applique notamment à la production de composés optiquement actifs, représentés par les formules générales : 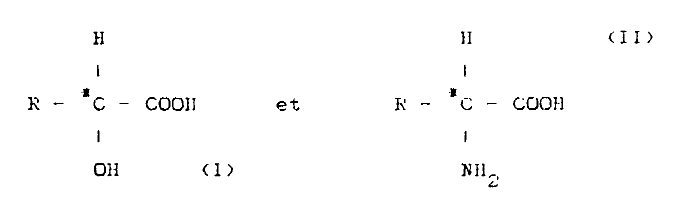
Lorsque l'on désire obtenir un composé de formule (I), on introduit comme substrat S, l'acide carboxylique α-cétonique correspondant (ou un sel de cet acide), et lorsque l'on désire obtenir un compose de formule (II), on introduit, comme substrat S, l'acide carboxylique α-cétonique correspondant (ou un sel de cet acide) en présence d'ammoniaque, les réductions conduisant à l'acide α alcool de formule (I) ou à l'acide α aminé de formule (II) s'effectuant selon les schémas réactionnels suivants : 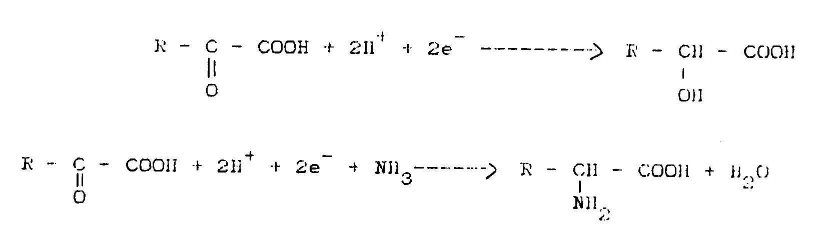
A titre d'exemples de composés que l'on peut préparer selon la présente invention, on peut citer l'acide lactique, l'acide malique, qui sont des composés de formule (I) - et leurs sels -, de nombreux acides α aminés, tels que l'α-alanine, qui sont des composés de formule (II).

La liste des composés que l'on peut préparer n'est pas exhaustive, la seule condition étant que l'on puisse associer les deux réactions antagonistes qui sont à la base de l'invention, à savoir la réduction électrochimique non stéréospécifique et l'oxydation enzymatique stéréospécifique, selon les critères suivants :
- disponibilité, ou possibilité d'induction dans un organisme vivant, de l'enzyme spécifique de l'isomère de P inverse de celui que l'on désire préparer ; et
- faisabilité de la réduction électrochimique de S dans des conditions physico-chimiques compatibles avec le fonctionnement de l'enzyme.

On peut introduire, comme enzyme, une déshydrogénase à co-substrat naturel comme NAD ou NADP, ou bien une oxydase, à co-substrat artificial (c'est-à-dire n'inter venant pas normalement dans les chaînes métaboliques), choisi notamment parmi les accepteurs d'electrons comme les quinones, le ferrocéne, le ferricyanure et des colorants, par exemple, le dichlorophénolindophénol.

On peut citer, a titre d'exemples particulier, les D- ou L-lactate déshydrogénases la L-malate déshydrogénase, la L-alanine déshydrogénase et les D- ou L-amino acides oxydases.

Selon l'invention, on conduit avantageusement la réaction en milieu solvant, notamment dans l'eau ou dans un solvant organique compatible avec le maintien de l'activite enzymatique, tout en permettant la fourniture de protons lors de la réduction, l'eau étant préférée. On peut également utiliser les mélanges eau-solvant organique.

L'enzyme peut être en solution dans le milieu réactionnel, ou bien être immobilisée sur des particules non proeuses ou poreuses, comme les mousses décrites dans l'article de G. Broun, O. Thomas, G. Gellf, D. Domurado, A.M. Berjonneau et C. Guillon, Biotechnology and Bioengineering 15, 359-375 (1973), ces particules baignant dans le milieu réactionnel, ou encore l'enzyme peut être immobilisée sur l'anode, avantageusement comme décrit dans C. Bourdillon, J.P. Bourgeois et D. Thomas, Journal of American Chemical Society, Vol 102, 1ère page 4231 (1980).

Dans le cas où l'on utilise, comme enzyme, une deshydrogénase à co-substrat NAD ou NADP, le pH du milieu peut se situer entre 6 et 10 (ce qui correspond à la zone de stabilité maximale pour les co-substrats NAD(H) et NADP(H) ; le potentiel de travail de l'anode, entre 0,5 et 0,9 V/électrode au calomel et KCl saturé (ECS ; et le potentiel de travail de la cathode, entre -0,7 et -1,5 V/ECS.

Le potentiel de travail de l'anode est choisi de telle sorte que seul le NADH soit oxydé. Ceci ne pose pas en général de problème car les groupements OH ou NH₂ des produits ne sont oxydables qu'à partir de 1,5 V/ECS.

Le choix du potentiel de fonctionnement de la cathode se pose dans les mêmes termes. La réduction parasite du NADH commence à partir de -0,9 V/ECS selon la réaction suivante :
2NAD⁺ + 2e⁻ → NAD-NAD (dimère enzymatiquement inactif)
Le potentiel de travail est choisi de telle sorte que la vitesse de réduction de S soit supérieure à celle de la dimérisation du NAD. Il est possible néanmoins de tolérer la formation de ce dimère car il se réoxyde aisément en monomère enzymatiquement actif à l'anode vers 0,2 V/ECS. Ceci augmente la consommation d'énergie, mais permet de réduire si nécessaire les substrats à des potentiels inférieurs à -0,9 V/ECS.

Dans le cas où l'on utilise, comme enzyme, une oxydase dont le co-substrat est un acceptuer d'électrons, le pH du milieu réactionnel peut se situer entre 4 et 8 ; le potentiel de travail de l'anode, entre 0,1 et 0,9 V/ECS ; et le potentiel de travail de la cathode entre -0,7 et -1,5 V/ECS.

Par ailleurs, selon l'invention, on peut utiliser une cathode réalisée en un matériau choisi parmi les métaux, par exemple le mercure et le nickel, et le carbone ; et une anode réalisée en un matériau choisi parmi le carbone et les métaux, par exemple les métaux nobles, comme le titane platiné. Les électrodes peuvent revêtir diverses formes.

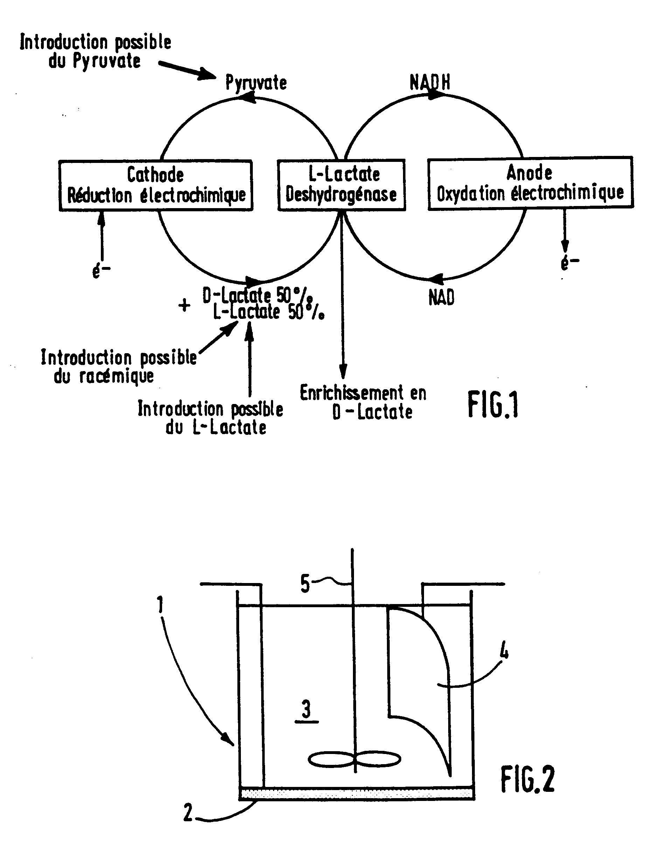

Sur la figure 1 du dessin annexé, on a représenté le schéma réactionnel résumant le procédé dans le cas d'utilisation d'une L-lactate déshydrogénase comme enzyme et du pyruvate comme substrat.

Les trois voies possibles d'utilisation du procédé apparaissent sur ce schéma, à savoir qu'en dehors de l'introduction de S et la transformation complète en D-P, on peut également ou bien introduire le racémique L/D-P et effectuer la résolution de ce racémique en L-P, ou bien introduire l'isomère L-P et effectuer son inversion de configuration.

Le bilan global correspond à la disparition de S et/ou de L-P. On consomme uniquement de l'énergie électrique et des quantités catalytiques de NAD et d'enzyme.

Sur la figure 2, on a représenté schématiquement un premier réacteur électrochimique 1 dans lequel le procédé de l'invention peut être mis en oeuvre d'une manière extrêmement simple. Ce réacteur 1 comporte un unique compartiment sur le fond duquel repose une nappe de mercure 2, utilisée comme cathode. Le compartiment est rempli d'une solution 3 contenant un tampon acide/base servant d'électrolyte, l'enzyme et le substrat à transformer. Dans la solution 3, plongent une anode 4 en carbone (ou en titane platiné), ainsi qu'un agitateur 5.

On conduit le procédé en discontinu en imposant une différence de potentiel entre les deux électrodes 2 et 4 jusqu'à totale transformation du substrat.

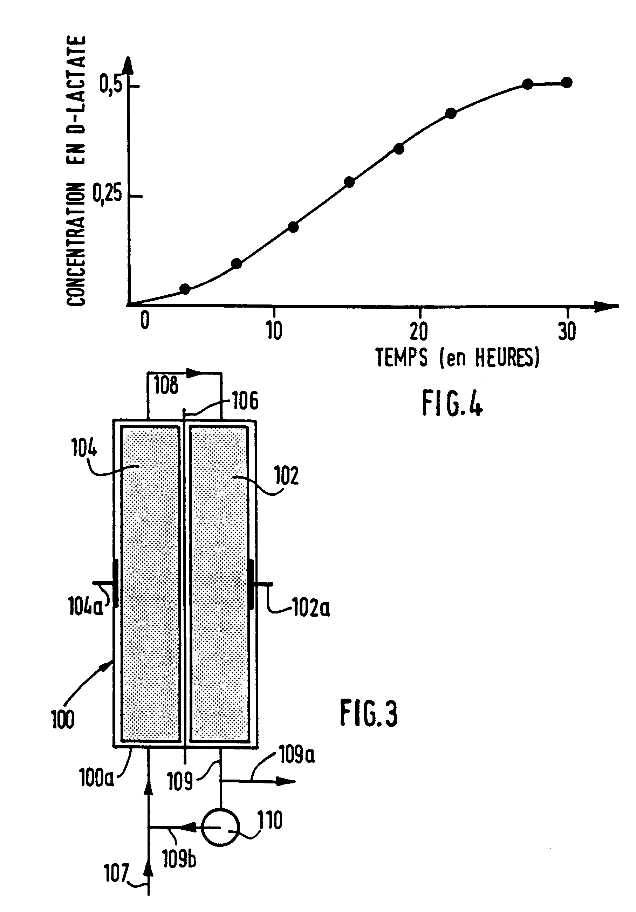

Sur la figure 3, on a représenté, schématiquement un second réacteur 100, à boucle de recyclage, pouvant également être utilisé pour la mise en oeuvre du procédé de l'invention.

Ce réacteur 100 comprend une enveloppe 100a renfermant une cathode 102, en feutre de carbone, et une anode 104, egalement en feutre de carbone ("RVG 4000", commercialisée par la Société "CARBONE LORRAINE"), sur laquelle l'enzyme utilisée est immobilisée (selon le protocole indiqué ci-après), cathode et anode étant séparées par une membrane échangeuse d'ions 106 servant de separateur entre les deux compartiments.

Chaque électrode est relié à un collecteur de courant, respectivement 102a et 104a.

La solution à traiter pénètre dans le compartiment anodique par la canalisation 107, elle est extraite de ce compartiment par la canalisation 108 et, de la, elle est renvoyée dans le compartiment cathodique 102 d'où elle est extraite par la canalisation 109, laquelle comporte une première dérivation 109a qui est la canalisation de sortie des produits et une dérivation 109b, qui est dérivation de recyclage dans la canalisation d'entrée 107, une pompe 110 de recirculation se situant sur le trajet de cette dérivation 109b.

Dans ce qui suit, on donnera deux exemples de mise en oeuvre du procédé selon la présente invention.

### Exemple 1 :

### Production de D-lactate à partir de pyruvate

Le schéma réactionnel est le suivant : 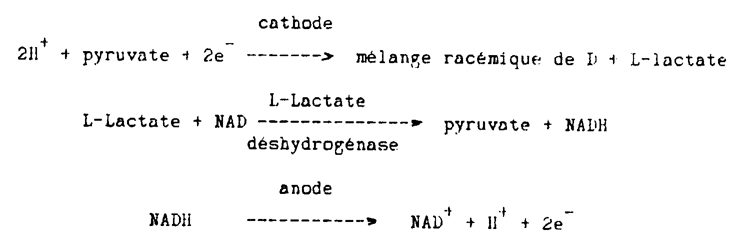
Dans un réacteur du type de celui représenté sur la figure 2, contenant 100 cm³ de tampon phosphate pH 8, 0,5M, 2 mg de L-lactate déshydrogénase (E.C.1.1.1.27.) (type 2 de la Société "SIGMA") et 7 mg de NAD, on introduit du pyruvate concentré (3M), avec un débit de 0,6 cm³/h.

Parallèlement, la différence de potentiel entre l'anode et la cathode est progressivement, en l'espace de 1/2 heure, portée à 1,8 Volt. L'état stationnaire du réacteur étant établi en 1 heure environ, l'alimentation en pyruvate et la différence de potentiel sont maintenues de telle sorte que la concentration en pyruvate soit toujours à 10⁻³M, pour limiter la réaction inverse de la déshydrogénase. La réaction est poursuivie pendant 30 heures et le courant décroît lentement de 240 à 140 mA. On cesse d'introduire du pyruvate une heure avant l'arrêt ou réacteur.

La figure 4 montre l'évolution de la concentration en D-lactate dans le réacteur, en fonction du temps. La concentration atteint 45 g/l en D-lactate à l'arrêt ou réacteur, dont moins de 1% de L-lactate et de pyruvate résiduels.

La transformation d'une mole de pyruvate en D-lactate consomme 4xF coulombs, dans les conditions expérimentales ci-dessus (réacteur alimenté)

### Exemple 2 :

### Production de D-lactate à partir du racémique D + L-lactate

On procède come indiqué à l'exemple 1, en introduisant du lactate racémique à la place du pyruvate. On obtient la même concentration en D-lactate qu'à l'exemple 1, en l'espace de 19 heures, avec des courants de l'ordre de 160 mA. Comme précédemment, le NAD a été régénéré environ 10 000 fois.

La transformation d'une mole de racémique consomme 2xF coulombs dans les conditions expérimentales précitées.

### Exemple 3 :

### Production en continu du D-malate à partir du mélange racémique D + L malate.

La schéma réactionnel est le suivant :

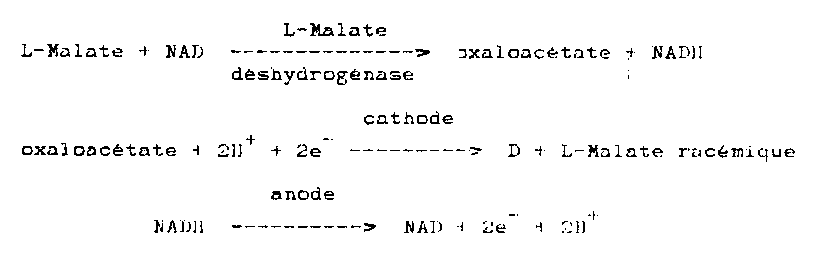

Ce procédé est mis en oeuvre avec un réacteur à solution percolante, du type de celui représenté sur la figure 3. L'immobilisation de l'enzyme est réalisée par greffage direct de la protéine sur l'électrode de régénération du NAD (anode). Cette configuration est particulièrement intéressante pour assurer le déplacement de l'équilibre thermodynamique, qui n'est pas en faveur de la formation d'oxaloacétate.

Méthode d'immobilisation de l'enzyme (selon la technique susmentionnée) :
Le feutre de carbone (graphite) constituant l'anode est tout d'abord oxydé superficiellement par traitement chimique à 105°C dans HNO₃ concentré. Il est ensuite monté dans le réacteur, puis rincé par circulation d'eau, jusqu'à pH neutre. On introduit 40 cm³ de la solution d'immobilisation dans le feutre, par circulation, puis on laisse au repos pendant 12 heures, afin que la polymérisation soit totale. La solution d'immobilisation contient 10 mg de L-malate déshydrogénase (E.C.1.1.1.37.) (fabriqué par la Société "SIGMA") + 10 mg de cyclohexyl-1-(morpholino-2 éthyl)-3 carbodiimide méthoxy p-toluène sulfonate (fabriqué par la Société "SIGMA") dans 40 cm³ de tampon phosphate, pH 7,5, 0,02 M.

Après rinçage pendant 1 heure à, l'aide du tampon de travail (tampon phosphate 0,5M, pH 8,5), le réacteur est prêt à l'emploi.

Le fonctionnement du réacteur en situation stationnaire correspond aux paramètres suivants :
- le volume de tampon de travail circulant la boucle du réacteur est de 100 cm³ ;
- le débit de la pompe de recyclage 110 est de 0,5 l/h ;
- le mélange racémique contenant 1 mole par litre de malate et 10⁻⁴ mole par litre de NAD dans le tampon de travail est introduit par la canalisation 107 avec un débit de 5 cm³/h ;
- la différence de potentiel entre l'anode et la cathode est maintenue à 1,7V et le courant résultant est d'environ 0,3 Ampère ;
- les concentration des espèces dans le réacteur à l'état stationnaire sont de 10⁻²M pour la somme oxaloacétate + L)-malate, 10⁻⁴ pour NAD + NADH, et 1M pour D-malate.

Ces concentrations sont celles obtenues dans la canalisation de sortie 109a. Le D-malate est pur à 1% prés dans cette expérience. Cette pureté peut être augmentée ou réduite par ajustement du débit de la pompe de recyclage 110.

## Revendications

1. Procédé électroenzymatique de production d'un composé de pureté énantiomérique contrôlée, respectivement D-P ou L-P, à partir d'un substrat S constitué par la forme oxydée du racémique correspondant D/L-P, ou dudit racémique D/L-P, ou encore de l'isomère optique inverse de celui que l'on veut préparer, caractérisé par le fait que l'on introduit, dans un réacteur électrochimique, respectivement ledit substrat S, ledit racémique D/L-P ou l'isomère optique inverse de celui que l'on veut préparer, ainsi qu'une enzyme oxydoréductase capable de catalyser l'oxydation de l'isomère optique inverse de celui que l'on veut préparer ; qu'on impose une différence de potentiel entre les électrodes, afin d'effectuer la réduction cathodique non stéréospécifique dudit substrat S, jusqu'à obtention de l'isomère ayant la pureté énantiomérique recherché ; et qu'on régénère le co-substrat de ladite enzyme par oxydation anodique dans ledit réacteur électrochimique.

2. Procédé électroenzymatique selon la revendication 1, caractérisé par le fait que l'on introduit, en tant que substrat S, acide carboxylique α-cétonique ou un acide carboxylique α-cétonique en présence d'ammoniaque.

3. Procédé électroenzymatique selon la revendication 1, caractérisé par le fait que l'on introduit, en tant que racémique D/L-P, un acide α alcool ou un acide α aminé.

4. Procédé électroenzymatique selon l'une des revendications 1 à 3, caractérisé par le fait que l'on introduit, en tant qu'enzyme, une déshydrogénase ou une oxydase.

5. Procédé électroenzymatique selon la revendication 4, caractérisé par le fait que l'on choisit une déshydrogénase dont le co-substrat est un co-substrat naturel de type Nicotinamide Adénine Dinucléotide ou Nicotinamide Adénine Dinucléotide Phosphate.

6. Procédé électroenzymatique selon la revendication 4, caractérisé par le fait que l'on choisit une oxydase dont le co-substrat est un co-substrat artificial, choisi notamment parmi les quinones, le ferrocéne, le ferricyanure et les colorants accepteurs d'électrons.

7. Procédé électroenzymatique selon l'une des revendications 1 à 6, caractérisé par le fait que l'on conduit la réaction en milieu solvant, notamment dans l'eau ou dans un solvant organique, ou dans un mélange d'un solvant organique avec l'eau, ledit solvant organique étant compatible avec le maintien de l'activité enzymatique, tout en permettant la fourniture de protons lors de la réduction.

8. Procédé électroenzymatique selon l'une des revendications 1 à 7, caractérisé par le fait que l'enzyme est en solution dans le milieu réactionnel.

9. Procédé électroenzymatique selon l'une des revendications 1 à 7, caractérisé par le fait que l'enzyme est immobilisée sur des particules poreuses ou non, baignant dans le milieu réactionnel.

10. Procédé électroenzymatique selon l'une des revendications 1 à 7, caractérisé par le fait que l'enzyme est immobilisée sur l'anode.

11. Procédé électroenzymatique selon l'une des revendications 1 à 10, suivant lequel on utilise, comme enzyme, une déshydrogénase dont le co-substrat est NAD ou NADP, caractérisé par le fait que le pH du milieu réactionnel se situe entre 6 et 10 ; le potentiel de travail de l'anode, entre 0,5 et 0,9 V/électrode au calomel et KCl saturé (ECS) et le potentiel de travail de la cathode, entre -0,7 et -1,5 V/ECS.

12. Procédé électroenzymatique selon l'une des revendications 1 à 10, suivant lequel on utilise, comme enzyme, une oxydase dont le co-substrat est un accepteur e'électrons, caractérisé par le fait que le pH du milieu réactionnel se situe entre 4 et 8 ; le potentiel de travail de l'anode, entre 0,1 et 0,9 V/ECS ; et le potentiel de travail de la cathode, entre -0,7 et 1,5 V/ECS.

13. Procédé électroenzymatique selon l'une des revendications 1 à 12 , caractérisé par le fait qu'on utilise une cathode réalisée en un matériau choisi parmi les métaux et le carbone, et une anode réalisée en un matériau choisi parmi le carbone et les métaux.

## Claims

1. Electroenzymatic process for producing a compound of controlled enantiomeric purity, D-P or L-P respectively, from a substrate S consisting of the oxidized form of the corresponding D/L-P racemate, or of the said D/L-P racemate, or else of the optical isomer which is the inverse of that which it is wished to prepare, characterized in that the said substrate S, the said D/L-P racemate or the optical isomer which is the inverse of that which it is wished to prepare, respectively, is introduced into an electrochemical reactor together with an oxidoreductase enzyme capable of catalyzing the oxidation of the optical isomer which is the inverse of that which it is wished to prepare; in that a potential difference is applied between the electrodes in order to perform the non stereospecific cathodic reduction of the said substrate S until the isomer having the required enantiomeric purity is obtained; and in that the cosubstrate of the said enzyme is regenerated by anodic oxidation in the said electrochemical reactor.

2. Electroenzymatic process according to claim 1, characterized in that an α-ketocarboxylic acid or an α-ketocarboxylic acid in the presence of aqueous ammonia is introduced as substrate S.

3. Electroenzymatic process according to claim 1, characterized in that an α-alcohol acid or an α-aminoacid is introduced as D/L-P racemate.

4. Electroenzymatic process according to one of claims 1 to 3, characterized in that a dehydrogenase or an oxidase is introduced as enzyme.

5. Electroenzymatic process according to claim 4, characterized in that a dehydrogenase is chosen whose cosubstrate is a natural cosubstrate of the type of nicotinamide adenine dinucleotide or nicotinamide adenine dinucleotide phosphate.

6. Electroenzymatic process according to claim 4, characterized in that an oxidase is chosen whose cosubstrate is an artificial cosubstrate chosen especially from quinones, ferrocene, ferricyanide and electron-acceptor dyes.

7. Electroenzymatic process according to one of claims 1 to 6, characterized in that the reaction is conducted in a solvent medium, especially in water or in an organic solvent, or in a mixture of an organic solvent with water, the said organic solvent being compatible with maintaining the enzyme activity while permitting the supply of protons during the reduction.

8. Electroenzymatic process according to one of claims 1 to 7, characterized in that the enzyme is in solution in the reaction medium.

9. Electroenzymatic process according to one of claims 1 to 7, characterized in that the enzyme is immobilized on porous or nonporous particles immersed in the reaction medium.

10. Electroenzymatic process according to one of claims 1 to 7, characterized in that the enzyme is immobilized on the anode.

11. Electroenzymatic process according to one of claims 1 to 10, according to which the enzyme employed is a dehydrogenase whose cosubstrate is NAD or NADP, characterized in that the pH of the reaction medium is between 6 and 10; the working potential of the anode, between 0.5 and 0.9 V/electrode containing calomel and saturated KCl (SCE) and the working potential of the cathode between -0.7 and -1.5 V/SCE.

12. Electroenzymatic process according to one of claims 1 to 10, according to which the enzyme employed is an oxidase whose cosubstrate is an electron acceptor, characterized in that the pH of the reaction medium is situated between 4 and 8; the working potential of the anode, between 0.1 and 0.9 V/SCE; and the working potential of the cathode, between -0.7 and -1.5 V/SCE.

13. Electroenzymatic process according to one of claims 1 to 12, characterized in that a cathode made of a material chosen from metals and carbon, and an anode made of a material chosen from carbon and metals, are employed.

## Patentansprüche

1. Elektroenzymatisches Verfahren zur Herstellung einer Verbindung von enantiomerisch kontrollierter Reinheit - entsprechend D-P oder L-P -, ausgehend von einem Substrat S, das aus der oxidierten Form des dem D/L-P entsprechenden Racemats oder aus dem Racemat D/L-P oder auch aus dem, dem herzustellenden entgegengesetzten optischen Isomeren besteht, dadurch gekennzeichnet, daß man in einen elektrochemischen Reaktor jeweils das Substrat S, das Racemat D/L-P oder das dem herzustellenden entgegengesetzte optische Isomer eingibt, ebenso wie ein Oxydoreduktase-Enzym, das befähigt ist die Oxidation des dem herzustellenden entgegengesetzten optischen Isomeren zu katalysieren, daß man eine Potentialdifferenz zwischen den Elektroden anlegt, um die kathodische, nicht stereospezifische Reduktion des Substrats S bis zum Erhalten des Isomeren mit der erwünschten enantiomerischen Reinheit durchzuführen, und daß man das Co-Substrat des Enzyms durch anodische Oxidation im elektrochemischen Reaktor regeneriert.

2. Elektroenzymatisches Verfahren nach Anspruch 1, dadurch gekennzeichnet, daß man als Substrat S eine α-Ketocarbonsäure oder eine α-Ketocarbonsäure in Gegenwart von Ammoniak verwendet.

3. Elektroenzymatisches Verfahren nach Anspruch 1, dadurch gekennzeichnet, daß man als D/L-P Racemat eine Säure mit einer α-ständigen Alkoholgruppe oder eine α-Aminosäure verwendet.

4. Elektroenzymatisches Verfahren nach einem der Ansprüche 1 bis 3, dadurch gekennzeichnet, daß man als Enzym eine Dehydrogenase oder eine Oxydase verwendet.

5. Elektroenzymatisches Verfahren nach Anspruch 4, dadurch gekennzeichnet, daß man eine Dehydrogenase auswählt, deren Co-Substrat ein natürliches Co-Substrat vom Typ Nicotinamid-Adenin-Dinucleotid oder Nicotinamid-Adenin-Dinucleotid-Phosphat ist.

6. Elektroenzymatisches Verfahren nach Anspruch 4, dadurch gekennzeichnet, daß man eine Oxydase auswählt, deren Co-Substrat ein künstliches Co-Substrat ist, das insbesondere aus Chinonen, Ferrocen, Ferricyanid und elektronenanziehenden Farbstoffen ausgewählt ist.

7. Elektroenzymatisches Verfahren nach einem der Ansprüche 1 bis 6, dadurch gekennzeichnet, daß man die Reaktion in einem Lösungsmittel, insbesondere in Wasser oder einem organischen Lösungsmittel oder in einer Mischung aus einem organischen Lösungsmittel und Wasser durchführt, wobei das organische Lösungsmittel die Beibehaltung der enzymatischen Aktivität gewährleistet und insgesamt die Versorgung mit Protonen bei der Reduktion gewährleistet ist.

8. Elektroenzymatisches Verfahren nach einem der Ansprüche 1 bis 7, dadurch gekennzeichnet, daß das Enzym in Lösung im Reaktionsmedium vorliegt.

9. Elektroenzymatisches Verfahren nach einem der Ansprüche 1 bis 7, dadurch gekennzeichnet, daß das Enzym auf porösen oder nicht porösen Teilchen, die im Reaktionsmedium schwimmen, immobilisiert ist.

10. Elektroenzymatisches Verfahren nach einem der Ansprüche 1 bis 7, dadurch gekennzeichnet, daß das Enzym auf der Anode immobilisiert ist.

11. Elektroenzymatische Verfahren nach einem der Ansprüche 1 bis 10, nach dem man als Enzym eine Dehydrogenase verwendet, deren Co-Substrat NAD oder NADP ist, dadurch gekennzeichnet, daß der pH-Wert des Reaktionsmediums zwischen 6 und 10, das Arbeitspotential der Anode zwischen 0,5 und 0,9 V/Kalomel- und gesättigter KCl-Elektrode (ECS) und das Arbeitspotential der Kathode zwischen -0,7 und -1,5 V/ECS beträgt.

12. Elektoenzymatisches Verfahren nach einem der Ansprüche 1 bis 10, nach dem man als Enzym eine Oxydase verwendet, deren Co-Substrat ein Elektronenakzeptor ist, dadurch gekennzeichnet, daß der pH-Wert des Reaktionsmediums zwischen 4 und 8, das Arbeitspotential der Anode zwischen 0,1 und 0,9 V/ECS und das Arbeitspotential der Kathode zwischen -0,7 und -1,5 V/ECS liegt.

13. Elektroenzymatisches Verfahren nach einem der Ansprüche 1 bis 12, dadurch gekennzeichnet, daß man eine Kathode verwendet, die aus einem Material besteht, das aus Metallen oder Kohlenstoff ausgewählt ist und daß man eine Anode verwendet, die aus einem Material besteht, das aus Kohlenstoff und Metallen ausgewählt ist.
